# EUROPEAN PATENT APPLICATION

(11) **EP 2 130 539 A1**
(43) Date of publication of application: **09.12.2009**
(21) Application number: 08722191.7
(22) Date of filing: 14.03.2008
(51) Int. Cl.: A61K 31/485, A61K 9/24, A61K 47/02, A61K 47/32, A61P 25/04, C07D 489/02, A61K 47/10

(54) **THERAPEUTIC TABLET FOR POSTHERPETIC NEURALGIA AND METHOD OF TREATING POSTHERPETIC NEURALGIA**

(30) Priority: 02.04.2007 JP 2007096812
(71) Applicant: Toyo Boseki Kabushiki Kaisha, Kita-ku, Osaka-shi Osaka 530-8230 (JP); FUSO PHARMACEUTICAL INDUSTRIES, LTD., Chuo-ku Osaka-shi Osaka 541-0045 (JP)
(72) Inventor: FUKUNO, Takuji, Ohtsu-shi Shiga 520-0292 (JP); TACHIMORI, Hiroshi, Ohtsu-shi Shiga 520-0292 (JP); MUKUNOKI, Fuminori, Ohtsu-shi Shiga 520-0292 (JP); MIYASAKA, Tadayo, Osaka-shi Osaka 530-8230 (JP); ARAKI, Hiromasa, deceased (JP); TANAKA, Shuichi, Osaka-shi Osaka 536-8523 (JP); DOI, Shingo, Osaka-shi Osaka 536-8523 (JP); KAWAZOE, Shoichi, Osaka-shi Osaka 536-8523 (JP)
(74) Representative: Vossius & Partner
(86) International application number: PCT/JP2008/054795
(87) International publication number: WO 2008/120562

(57) **Abstract**

The present invention provides a tablet for treating postherpetic neuralgia and a method of treating postherpetic neuralgia with the use of the tablet. The therapeutic tablet for postherpetic neuralgia according to the present invention is **characterized in** comprising buprenorphine hydrochloride, having a double layer structure consisting of a quick-release layer and a sustained-release layer, wherein the tablet is adhesive to the oral mucosa.

## Description

### TECHNICAL FIELD

The present invention relates to a tablet for treating postherpetic neuralgia and a method of treating postherpetic neuralgia with the use of the tablet.

### BACKGROUND ART

"Pain" includes various kinds ranging from acute pain to chronic pain, such as inflammatory pain due to inflammation, nociceptive pain caused by cancer and the like, and neuropathic pain (see Non-Patent Document 1). Among the pains, the neuropathic pain is a generic term used to refer to pains developed by the damage, compression and the like of nerve tissues; and is accompanied by severe discomfort and unusual sense such as numbness. However, the cause of developing neuropathic pain is not well known, and adequate therapy therefor is not established.

For example, it is disclosed in Non-Patent Document 1 that methods of treating inflammatory pain and nociceptive pain have been almost established, while development of therapeutic agent for chronic pains such as neuropathic pains is just begun. Non-Patent Document 1 was just published in 2006; and it can be thought from the fact that the development of therapeutic means for neuropathic pain has still not been adequately developed.

The neuropathic pain include not only postherpetic neuralgia and complex regional pain syndrome but also phantom limb pain that is felt in amputated limbs, and the cause is not sufficiently clarified.

Among neuropathic pain, postherpetic neuralgia is a severe pain developed after treatment of herpes zoster. The mechanism of developing the severe pain developed after treatment of herpes zoster is not completely revealed; but, it is said that viruses still remaining in ganglia even after treatment of herpes zoster are reactivated due to immunological deterioration or the like, and act directly on nerves to cause postherpetic pain. Accordingly, postherpetic neuralgia is problematic particularly in the elderly inferior in immunity. Some data reported that several dozen % of adults remain infected with herpes zoster virus even after treatment of herpes zoster; therefore, positive treatment of postherpetic neuralgia becomes important as the population is aging, along with early treatment of herpes zoster.

It is said that complete treatment is difficult after transition from herpes zoster to postherpetic neuralgia. For example, a non-steroidal analgesic agent is first administered in many cases in actual treatment of postherpetic neuralgia; but, the possibility of thereby attaining complete responses is very low. Physical methods such as acupuncture treatment and low-frequency stimulation treatment are used in some cases, but cannot serve as fundamental therapies. A method of directly alleviating the pain with local anesthetic agents is sometimes used, but is not effective since the site where the pain of postherpetic neuralgia develops is often not clear and agents for external use, such as local anesthetic agent, cannot reach the deep portion of skin.

It is described in Non-Patent Document 1 that amitriptyline and mexiletine are used for postherpetic neuralgia. However, the drugs are originally an antidepressant drug and an antiarrhythmic drug, respectively. Thus, there is no drug specified in treatment of postherpetic neuralgia under the present circumstances.

It is described in Non-Patent Documents 2 and 3 that Neurotropin^{™}, which is an extract from the inflammatory skin of vaccinia virus-inoculated rabbit, exerts a certain effect on postherpetic neuralgia. However, the inhibitory action of Neurotropin on postherpetic neuralgia is not significantly different 2 weeks after administration relative to a placebo group, and becomes significantly different barely 4 weeks after administration, as shown in Non-Patent Document 2. Further, the inhibitory action of Neurotropin on postherpetic neuralgia does not significantly differ from that before administration of the drug according to an experiment disclosed in Non-Patent Document 3.

In Patent Document 1, is disclosed a method of using devazepide in combination with an opioid analgesic agent in order to reduce the dose of the opioid analgesic agent. In the document, is also described a clinical example where devazepide together with an opioid analgesic agent such as morphine and, if necessary, together with an analgesic agent such as buprenorphine for sudden pain, was administered by the patient with neuropathic pain. However, the main objective of the invention described in Patent Document 1 lies in reducing the dose of an opioid analgesic agent by using devazepide in combination. In addition, it was only 10 of 41 subjects who attained pain relief by the invention.

In Non-Patent Document 4, is described a clinical example wherein an opioid analgesic agent such as oxycodone hydrochloride or morphine sulfate was intravenously dripped into the patient with postherpetic neuralgia. In Non-Patent Document 5, is also described a clinical example wherein oxycodone was intravenously dripped into the patient with postherpetic neuralgia. In Non-Patent Document 6, is described a clinical example wherein lidocaine and morphine were intravenously injected into the patient with postherpetic neuralgia.

In Patent Document 2, is disclosed a therapeutic agent for neuropathic pain containing an opioid receptor antagonist as an active ingredient; and buprenorphine is exemplified as the opioid receptor antagonist and postherpetic neuralgia is exemplified as neuropathic pain. However, there is no description of a specific example wherein buprenorphine was administered to the patient with postherpetic neuralgia therein.

Buprenorphine has been used as an analgesic agent and already marketed as an injection, a suppository and a sublingual tablet. However, the buprenorphine level in blood is rapidly increased and immediately decreased when the preparations are administered; and thus, the effect by the preparations is transient. Accordingly, regarding the preparations, highly frequent administration is necessary against chronic pain and may cause the problem of side effects. In sublingual tablets, there is also a problem of the poor absorption efficiency of buprenorphine itself. There is also an example where buprenorphine was used as a drug for external use; but the preparation is not practical since buprenorphine is extremely poor in percutaneous absorption.

The researcher belonging in the present applicant developed an oral mucosa-adhering buprenorphine preparation used mainly in treatment of cancer pain, and had a patent application on the preparation. The preparation is disclosed in Patent Document 3. However, the cancer pain is a nociceptive pain that is caused due to compression of an organ by growth of cancer cells, metastasis of cancer cell to bone, or a side effect of an anticancer agent; and the cause of thereof is completely different from that of neuropathic pain such as postherpetic neuralgia. Actually, major uses of a buprenorphine injection and suppository manufactured and marketed in Japan lie in relieving postsurgical pain, or pain attributable to cancer or myocardial infarction as well as in assisting anesthesia.
Non-Patent Document 1: Katsuo Toide, Journal of the Japanese Pharmacological Society, Vol.128, pp.321-325 (2006)
Non-Patent Document 2: Hideo Yamamura, et al. "Igaku No Ayumi" (Development of Medicine), Vol.147, No.7, pp.651-664 (1988)
Non-Patent Document 3: Michio Hashikabe, et al., Nishi Nichi Hifu (The Nishinihon Journal of Dermatology), Vo.65, No.1, pp.65-69 (2003)
Patent Document 1: Published Japanese translation of PCT international publication for patent application No.2005-533046
Non-Patent Document 4: Robert H. Dworkin et al., Archives of Neurology, Vol.60, pp.1524-1534 (2003)
Non-Patent Document 5: C. Peter N. Watson et al., American Academy of Neurology, Vol.50, pp.1837-1841 (1998)
Non-Patent Document 6: Michael C. Rowbotham et al., Neurology, Vol.41, pp.1024-1028 (1991)
Patent Document 2: Japanese Patent Publication No.2006-131545A
Patent Document 3: Japanese Patent Publication No.8-291070A

### DISCLOSURE OF THE INVENTION

As described above, postherpetic neuralgia becomes particularly problematic for the arrival of an aging society; however, there is still no effective therapeutic means therefor.

The objective of the present invention is to provide a practical therapeutic agent for postherpetic neuralgia and a method of treating postherpetic neuralgia.

The inventors searched for a drug capable of effectively treating postherpetic neuralgia. As a result, the inventors found that buprenorphine hydrochloride exhibits an extremely excellent therapeutic effect on postherpetic neuralgia when administered sustainably via oral mucosa, not through experiments on animals or the like, but through actual clinical tests; and the present invention was thereby completed.

The therapeutic tablet for postherpetic neuralgia according to the present invention is characterized in comprising buprenorphine hydrochloride, having a double layer structure consisting of a quick-release layer and a sustained-release layer, wherein the tablet is adhesive to the oral mucosa.

The method of treating postherpetic neuralgia according to the present invention is characterized in comprising a step of applying the above therapeutic tablet for postherpetic neuralgia according to the present invention on the oral mucosa of a patient.

### BRIEF DESCRIPTION OF THE DRAWINGS

FIG. 1 is a graph showing "pain VAS values" in case that a buprenorphine hydrochloride preparation was administered to the patient with complex regional pain syndrome.
FIG. 2 is a graph showing "pain VAS values" in case that a buprenorphine hydrochloride preparation was administered to the patient with postherpetic neuralgia.
FIG. 3 is a graph showing "pain degree (VRS)" in case that a buprenorphine hydrochloride preparation was administered to the patient with complex regional pain syndrome.
FIG. 4 is a graph showing "pain degree (VRS)" in case that a buprenorphine hydrochloride preparation was administered to the patient with postherpetic neuralgia.

### BEST MODE FOR CARRYING OUT THE INVENTION

The therapeutic tablet for postherpetic neuralgia according to the present invention is characterized in comprising buprenorphine hydrochloride, having a double layer structure consisting of a quick-release layer and a sustained-release layer, wherein the tablet is adhesive to the oral mucosa.

The therapeutic objective of the tablet according to the present invention is postherpetic neuralgia. The cause of postherpetic neuralgia is not necessarily evident; however, postherpetic neuralgia is considered attributable to the virus which remains in ganglia even after treatment of herpes zoster and is reactivated due to reduction in immunity or the like and acts directly on nerves. Therefore, severe pain may persist for a long period, particularly in immune-compromised aged persons and sick persons. At the same time, the pain is very difficult to be reduced since the reactivated herpes zoster virus acts directly on nerves. Postherpetic neuralgia, which is hardly treatable by the prior art as described above, can be effectively cured with the therapeutic agent of the present invention.

The chemical name of buprenorphine hydrochloride is N-cyclopropylmethyl-7α-(S-1-hydroxy-1,2,2-trimethylpropyl)-6,1 4-endo-ethano-6,7,8,14-tetrahydronororipavine hydrochloride. Buprenorphine hydrochloride is used mainly as a nonnarcotic analgesic; and more specifically, is used in relieving pain after surgery or attributable to cancer and in assisting anesthesia.

Buprenorphine hydrochloride is preferably added in the tablet of the present invention in an amount of 0.1 mg or more per tablet. According to the inventors' finding, the inhibitory effect of the tablet on pain is increased with an increasing amount of buprenorphine hydrochloride per tablet. Even a tablet containing 0.1 mg of buprenorphine hydrochloride has an inhibitory effect on pain; but the effect is improved in a dose-dependent manner, and a tablet containing 0.3 mg of buprenorphine hydrochloride has a sufficient effect as shown in the later described Examples. At the same time, as the amount of buprenorphine hydrochloride per tablet increases, there is an increasing danger of adverse events such as side effects. Hence, the amount of buprenorphine hydrochloride per tablet is preferably 1.0 mg or less, more preferably 0.8 mg or less.

The tablet of the present invention has a double layer structure consisting of a quick-release layer and a sustained-release layer, and is used by being applied on the oral mucosa. Conventionally, buprenorphine hydrochloride preparations have been developed mainly as an injection or a suppository. In the present invention, however, buprenorphine is absorbed via the oral mucosa, thereby effectively suppressing postherpetic neuralgia. The tablet of the present invention has a double layer structure consisting of a quick-release layer and a sustained-release layer, thereby attaining both quick effect and sustained release.

The reason why postherpetic neuralgia is effectively suppressed by using such administration form is not necessarily evident. However, it can be estimated that, for example, the oral mucosa-adhering preparation having such a double layer structure does not cause rapid increase of the buprenorphine level in blood as compared with an injection and thus is less danger of side effects as well as excellent in durability of drug action. Although the site where the pain is felt cannot be accurately grasped in postherpetic neuralgia, buprenorphine absorbed moderately via the oral mucosa is likely of persistently stabilizing patients' feel, thereby making the patient forget the pain.

The tablet of the present invention can be produced by a conventional method. For example, the quick-release layer should be relatively rapidly disintegrated to release buprenorphine hydrochloride; therefore, relatively large amounts of a component used as excipients and disintegrators such as mannitol and talc is added in the layer.

It is preferable to add not only buprenorphine hydrochloride but also polyvinylpyrrolidone or a pharmaceutically acceptable salt thereof, polyacrylic acid or a pharmaceutically acceptable salt thereof, and sodium bicarbonate in the sustained-release layer of the tablet according to the present invention.

A sustained-release property is given to the sustained-release layer in the tablet of the present invention by adding both of polyvinylpyrrolidone or a pharmaceutically acceptable salt thereof and polyacrylic acid or a pharmaceutically acceptable salt therein. Specifically, polyvinylpyrrolidone having binding property and polyacrylic acid having adhesive property are allowed to be coexistent, so that suitable sustained-release property can be given to the sustained-release layer. Though the ratio of the two components may be appropriately regulated, the ratio of the polyvinylpyrrolidone or pharmaceutically acceptable salt thereof relative to the total of the polyvinylpyrrolidone or pharmaceutically acceptable salt thereof and the polyacrylic acid or pharmaceutically acceptable salt is preferably 5% by mass or more and 95% by mass or less. When the ratio is lower than 5% by mass, the expansibility of the tablet is so strong that foreign-body sensation may be caused upon application to the oral cavity. On the other hand, when the ratio is higher than 95% by mass, the adhesiveness of the tablet may be decreased.

It is preferable to add sodium bicarbonate in the sustained-release layer of the tablet according to the present invention. Buprenorphine hydrochloride as the active ingredient of the tablet according to the present invention is an acidic drug, and the swelling ability of polyacrylic acid may be deteriorated under acidic conditions. Therefore, a pH regulator, preferably sodium bicarbonate, is added to neutralize the sustained-release layer. The amount ratio of sodium bicarbonate added into the sustained-release layer is preferably 7.0% by mass or more and 7.5% by mass or less relative to the polyacrylic acid or pharmaceutically acceptable salt thereof. When the ratio is 7.0% by mass or more, the swelling ability of polyacrylic acid can be sufficiently secured, thereby more reliably releasing buprenorphine hydrochloride. On the other hand, when the ratio is higher than 7.5% by mass, a carboxy vinyl polymer is swollen so significantly that buprenorphine hydrochloride may be possibly released too quickly.

Examples of pharmaceutically acceptable salts of polyvinylpyrrolidone and polyacrylic acid include alkali metal salts such as sodium salt and potassium salt, alkaline earth metal salts such as calcium salt and magnesium salt, and ammonium salts.

It is possible to further add known compounding ingredients such as a lubricant, a binder, a flavoring substance and a coloring agent in the tablet of the present invention.

The tablet of the present invention can be produced, for example, by feeding ingredients for the quick-release layer and the sustained-release layer to a tableting machine capable of producing a double-layer tablet, and tableting the ingredients into double-layer tablets.

The method of treating postherpetic neuralgia according to the present invention is characterized in comprising a step of applying the above therapeutic tablet for postherpetic neuralgia according to the present invention on the oral mucosa of a patient. The dose of the tablet of the present invention may be controlled appropriately depending on, for example, the severity, age and sex of a patient; for example, one tablet may be applied once or twice per day on the gingival area.

### EXAMPLES

Hereinafter, the present invention is described in more detail with reference to the Examples; but the present invention is not limited to the Examples and can be carried out in such a range as to be adapted to the purport of the description in the specification; and any of such modifications are included in the scope of the present invention.

### Production Example 1: Production of an oral mucosa-adhering buprenorphine preparation

Buprenorphine hydrochloride (2.156 g), polyvinylpyrrolidone (27.4 g) and Edible Blue No. 1 (0.03 g) were dissolved in purified water (431.2 g). The resulting solution was sprayed onto D-mannitol (723.0 g) in a fluidized-bed granulating machine using purified water (50 g) as a rinse, and then dried, thereby preparing granules. The granules (100 parts by mass) were mixed with magnesium stearate (1 part by mass) and talc (0.56 part by mass), to prepare quick-release layer granules.

Separately, buprenorphine hydrochloride (4.312 g) and polyvinylpyrrolidone (8.624 g) were dissolved in purified water (862.4 g). The resulting solution was sprayed onto polyvinylpyrrolidone (844.9 g) in a fluidized-bed granulating machine using purified water (50 g) as a rinse, and then dried, thereby preparing granules. The granules (100 parts by mass) were mixed with polyacrylic acid (19.3 parts by mass) and sodium bicarbonate (1.42 parts by mass). The resulting mixture was compressed with a roller compactor. The compressed product was broken to pieces. Particle size regulation was carried out by using with a power mill; further, the particles were sieved with a sieve having 500-µm openings, to prepare sustained-release layer granules. The ratio of polyvinylpyrrolidone relative to the total of polyvinylpyrrolidone and polyacrylic acid in the sustained-release layer was about 83.8% by mass, and the ratio of sodium bicarbonate relative to polyacrylic acid in the sustained-release layer was about 7.4% by mass.

The quick-release layer granules and the sustained-release layer granules were charged into a tableting machine and formed into oral mucosa-adhering double-layer tablet each containing buprenorphine hydrochloride in amounts of 0.1 mg and 0.2 mg in the quick-release layer and sustained-release layer, respectively. Separately, buprenorphine hydrochloride was used in half or twice amount in the protocol described above, so oral mucosa-adhering double-layer tablet each containing buprenorphine hydrochloride in amounts of 0.05 mg and 0.1 mg in the quick-release layer and sustained-release layer respectively, or each containing buprenorphine hydrochloride in amounts of 0.2 mg and 0.4 mg in the quick-release layer and sustained-release layer respectively, were produced. Hereinafter, the buprenorphine hydrochloride tablets are referred to as 0.3 mg tablet, 0.15 mg tablet and 0.6 mg tablet, respectively. In addition, placebo tablet that did not contain buprenorphine hydrochloride but had the same appearance as that of the buprenorphine hydrochloride tablets were produced.

### Test Example 1

### (1) Double-Blind Comparative Test

First, a double-blind comparative test (hereinafter, referred to as "DB test") was carried out. The subjects in the test were specifically patients with postherpetic neuralgia and patients with complex regional pain syndrome, who were 20- to 74-year-old Asians out of patients with non-cancerous chronic pain and from whom the consent for the test was beforehand obtained. The pain therapy previously conducted up to 4 days before initiation of the test was continued, and the test was carried out in addition to the pain therapy. However, it was prohibited to add new pain therapy other than the therapy in the experiment and to change the condition of the previous pain therapy. The 126 subjects were divided at random into a group including 33 subjects given the placebo tablet , a group including 30 subjects given the 0.15 mg tablet, a group including 31 subjects given the 0.3 mg tablet and a group including 32 subjects given the 0.6 mg tablet.

First, the subjects were observed for 1 to 4 days without being administered with the tablets. The period is referred as "Former observation period". Then, the subjects were administered with the tablet for 7 days from the fifth day. The period is referred as "Administration period". In the administration period, the subjects were not informed of the dose. The administration was carried out once per day by inserting each tablet into the space between the upper lip and the upper gingival and then applying the sustained-release layer on the upper gingival. During the former observation period and the administration period, the intensity of pain, i.e. pain VAS value, was indicated at each point of time by the subjects with 100 mm as "maximum pain" and 0 mm as "no pain". At each point of time, the degree of pain, i.e. VRS, was evaluated by the subjects in 4 grades, that is, 0: no pain, 1: light, 2: moderate and 3: severe. During the test period, sleep, appetite, mood and pleasure were evaluated respectively in 4 grades from 0 to 3 by each subject, thereby determining "the degree of satisfaction of daily life".

During the test, 13 of 126 cases could not be evaluated for reasons such as failure to comply with provisions of the protocol. In the 113 cases that could be evaluated, the pain was hardly improved in the group given the placebo tablet, while the other groups were observed to get the effect to relieve pain in accordance with the dose of buprenorphine hydrochloride.

### (2) Continuous Administration Test

Out of the patients having completed the DB test, the patients for whom a principal investigator had judged the continuous administration to be beneficial and who had desired continuous administration were further subjected to a continuous administration test from 1 day after the DB test. The subjects who could be evaluated were made up of 20 patients with postherpetic neuralgia and 23 patients with complex regional pain syndrome.

The tablets containing buprenorphine hydrochloride in amounts of 0.3 mg and 0. 6 mg respectively, which were prepared in Production Example 1, were administered to the subjects in the same manner as described above in the DB test. Hereinafter, the tablets were referred to as "0.3 mg tablet" and "0.6 mg tablet". First, the 0.3 mg tablet was administered once per day to each patient. Then, the tablet was changed if necessary to the 0.6 mg tablet while the analgesic effect and safety were confirmed, so that the analgesic effect could persist for 24 hours. When the 0.6 mg tablet did not maintain the analgesic effect for 24 hours or when the 0.6 mg tablet should be changed to the 0.3 mg tablet for reasons such as adverse events, the tablet was administered twice per day, that is, at 12-hour intervals.

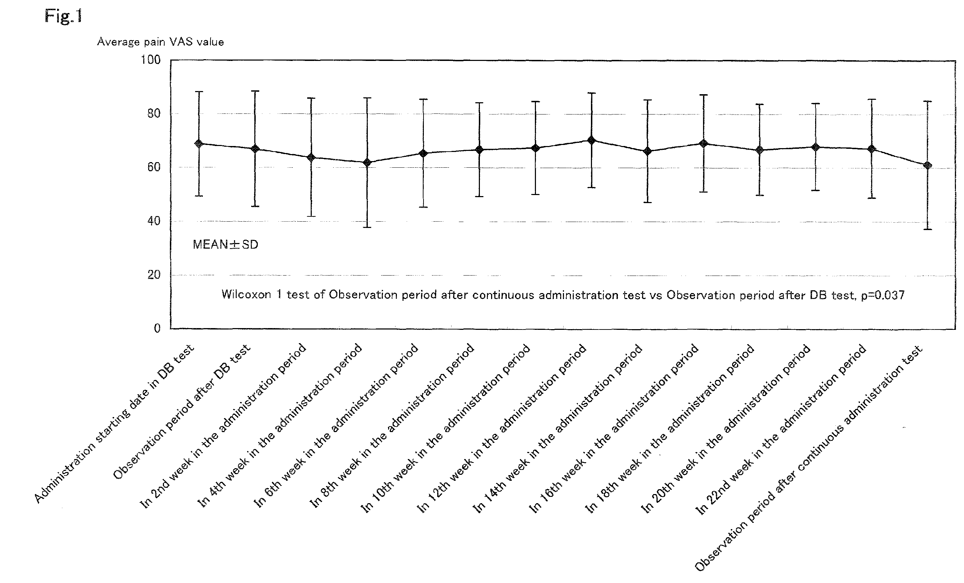

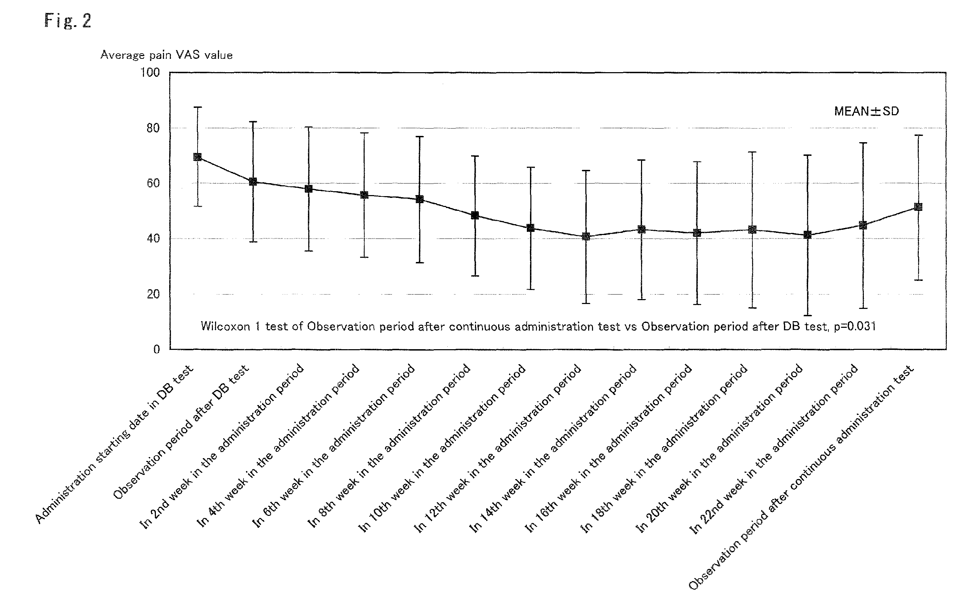

During the administration period, pain VAS value and pain degree, i.e. VRS, were evaluated by each subject in the same manner as in the DB test. The result of pain VAS value in the patients with complex regional pain syndrome is shown in Fig. 1, and the result of pain VAS value in the patients with postherpetic neuralgia is shown in Fig. 2. In Figs. 1 and 2, "Administration starting date in DB test" shows the result on the day when the administration of the tablet in the DB test was started, "Observation period after DB test" shows the result for 1 day after the DB test and before the continuous administration test, "In X week in the administration period" shows the result in X week from the start of administration of the buprenorphine hydrochloride tablet in the continuous administration test, and "Observation period after continuous administration test" shows the result on the day after the administration of the buprenorphine hydrochloride tablet over 24 weeks was finished.

As shown in Fig. 1, the VAS value was decreased from 67.0 ± 21.6 in the observation period after the DB test to 61.1 ± 23.9 in the observation period after the continuous administration test. The result was verified by Wilcoxon 1 sample test; as a result, there was a significant pain relief effect with p < 0.05 (p = 0.037). However, it can be seen from Fig. 1 that the VAS value does not change so much as a whole in case that the buprenorphine hydrochloride tablets were administered to the patients with complex regional pain syndrome.

On the other hand, it is shown in Fig. 2 that the VAS value was continuously decreased after start of the administration, so that the average VAS value that was nearly 70 on the starting date of administration was changed to remain stably in the 40s during the administration period, in case that the buprenorphine tablet was administered to the patients with postherpetic neuralgia. In other words, the patients with postherpetic neuralgia given the buprenorphine hydrochloride tablet felt that the pain was decreased to a degree less than the medium degree. The significant difference in the patients with postherpetic neuralgia in the observation period after the continuous administration test relative to the observation period after the DB test was verified by Wilcoxon 1 sample assay; as a result, the VAS value was decreased from 60.5 ± 21.8 to 51.3 ± 26.2, and there was a significant pain relief effect with p < 0.05 (p = 0.031).

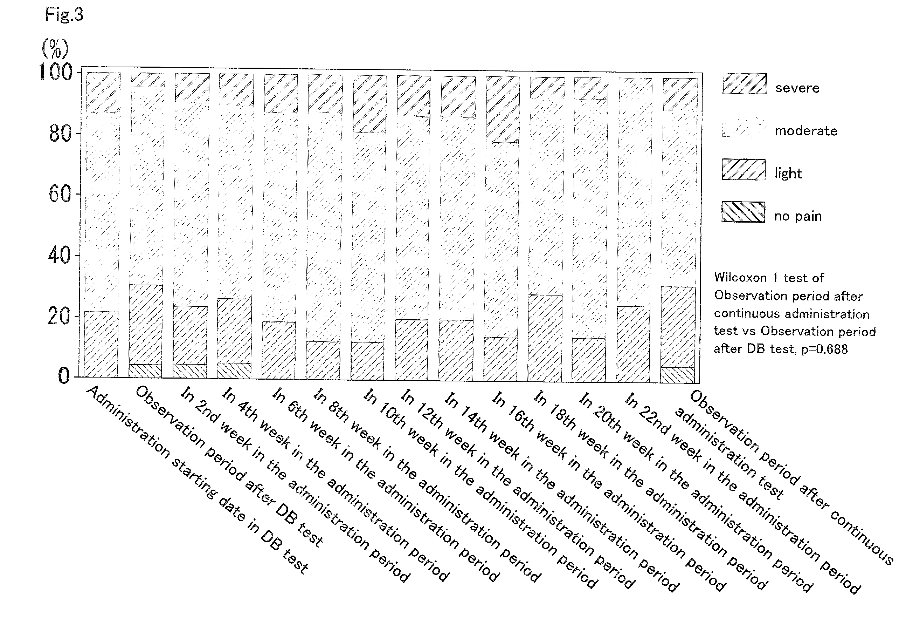

When the buprenorphine hydrochloride tablet was administered to the patients with complex regional pain syndrome, many of the patients answered "they can have a sound sleep" and the improvement could be shown. However, the other item "the degree of satisfaction of daily life" was not found to significantly change as a whole. As shown in Fig. 3, there was no improvement in "pain degree (VRS)".

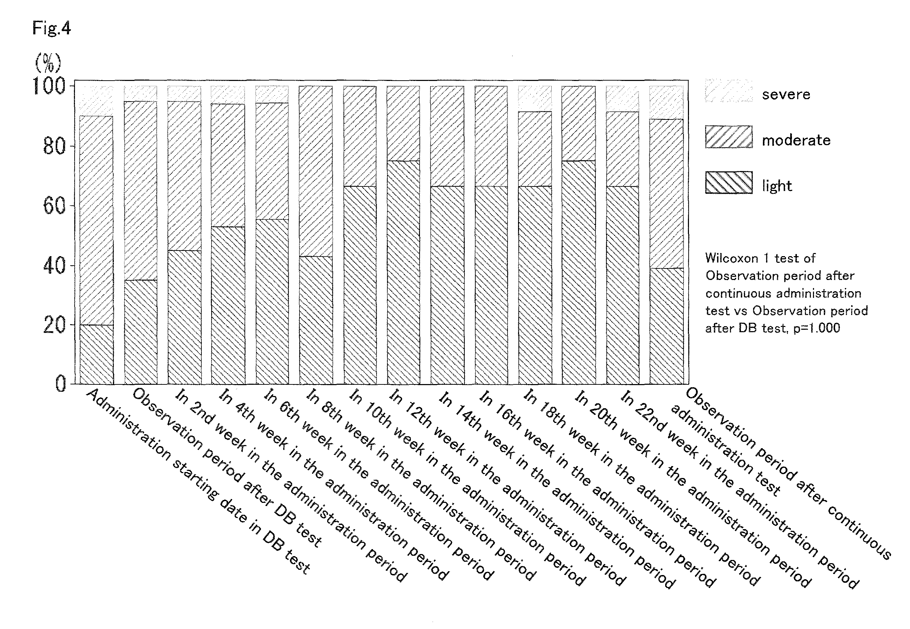

On the other hand, as shown in Fig. 4, sthe ratio of "1: light" in pain degree (VRS) was high during the administration period and thus the clear improvement could be shown, in a case the buprenorphine hydrochloride tablet was administered to the patients with postherpetic neuralgia. With respect to "the degree of satisfaction of daily life" in the patients with postherpetic neuralgia, a high proportion of the patients given the buprenorphine hydrochloride tablet answered "they can have a sound sleep", "feel very well", "feel well" and "are very enjoyable", and thus clear improvement tendency could be shown.

As shown in the results, it was demonstrated that the pharmaceutical preparation containing buprenorphine hydrochloride has an extremely excellent therapeutic effect on particularly postherpetic neuralgia among neuropathic pains.

### INDUSTRIAL APPLICABILITY

The therapeutic tablet for postherpetic neuralgia according to the present invention can very effectively cure postherpetic neuralgia for which there has been no truly effective therapeutic means. Accordingly, the tablet of the present invention is extremely industrially useful as a therapeutic agent for postherpetic neuralgia from which particularly the elderly suffers over a long period.

## Claims

1. A therapeutic tablet for postherpetic neuralgia,
comprising buprenorphine hydrochloride,
having a double layer structure consisting of a quick-release layer and a sustained-release layer,
wherein the tablet is adhesive to the oral mucosa.

2. The therapeutic tablet for postherpetic neuralgia according to claim 1, wherein the sustained-release layer contains buprenorphine hydrochloride, polyvinylpyrrolidone or a pharmaceutically acceptable salt thereof, polyacrylic acid or a pharmaceutically acceptable salt thereof, and sodium bicarbonate.

3. The therapeutic tablet for postherpetic neuralgia according to claim 1 or 2, wherein the ratio of the polyvinylpyrrolidone or pharmaceutically acceptable salt thereof relative to the total amount of the polyvinylpyrrolidone or pharmaceutically acceptable salt thereof and the polyacrylic acid or pharmaceutically acceptable salt in the sustained-release layer is 5% by mass or more and 95% by mass or less.

4. The therapeutic tablet for postherpetic neuralgia according to any one of claims 1 to 3, wherein the ratio of the sodium bicarbonate relative to the polyacrylic acid or pharmaceutically acceptable salt thereof in the sustained-release layer is 7.0% by mass or more and 7.5% by mass or less.

5. A method of treating postherpetic neuralgia, comprising a step of applying the therapeutic tablet for postherpetic neuralgia according to any one of claims 1 to 4 on the oral mucosa of a patient.
